Europäisches Patentamt

European Patent Office

Office européen des brevets

(11) Publication number: **0 150 176**
**B1**

# EUROPEAN PATENT SPECIFICATION

(45) Date of publication of patent specification: **07.09.88**

(21) Application number: **83902395.9**

(22) Date of filing: **29.06.83**

(86) International application number:
**PCT/US83/00994**

(87) International publication number:
**WO 85/00099 17.01.85 Gazette 85/02**

(51) Int. Cl.⁴: **A 61 B 5/02,** G 06 F 15/20

(54) **BLOOD PRESSURE MEASUREMENT WITH KOROTKOV SOUND ARTIFACT INFORMATION DETECTION AND REJECTION.**

(43) Date of publication of application:
**07.08.85 Bulletin 85/32**

(45) Publication of the grant of the patent:
**07.09.88 Bulletin 88/36**

(84) Designated Contracting States:
**DE FR GB NL**

(56) References cited:
**EP-A-0 029 349**
**DE-A-2 811 362**
**US-A-3 773 033**
**US-A-4 408 614**

Conference held at Stanford University in September 1978, publ. by Soc. of Photo-Opt. Instrumentation Engineers, C.S. Weaver et al.: "A study of noninvasive blood pressure measurement techniques", p. 99-105

Biomedizinische Technik, vol. 26, no. 4, April 1981 (Berlin, DE), B. Bertram et al.: "Automatische indirekte Blutdruckmessung in Ruhe und bei Belastung: Formanalyse der Korotkov-Geräusche; Variables Zeitfenster zur Artefaktunterdrückung", p. 81-84

(73) Proprietor: **SRI INTERNATIONAL**
**333 Ravenswood Avenue**
**Menlo Park, California 94025 (US)**

(72) Inventor: **WEAVER, Charles, Sinclair**
**336 Iris Way**
**Palo Alto, CA 94303 (US)**
Inventor: **CHITTENDEN Constance Thias**
**1621 Havenshurst Drive**
**Los Altos, CA 94022 (US)**

(74) Representative: **Müller, Hans-Jürgen, Dipl.-Ing. et al**
**Müller, Schupfner & Gauger Lucile-Grahn-Strasse 38 Postfach 80 13 69**
**D-8000 München 80 (DE)**

(56) References cited:
**Medical & Biological Engineering & Computing, vol. 19, no. 5, September 1981 (Stevenage, Herts, GB), L.A. GEDDES et al.: "Pulse arrival time as a method of obtaining systolic and diastolic blood pressure indirectly", p. 671-672**

Courier Press, Leamington Spa, England.

## Description

The invention concerns a method and an apparatus for identifying and removing artifacts from Korotov sound signals.

Numerous means for obtaining blood pressure measurements are known including both invasive and noninvasive systems. For instance a noninvasive system is disclosed in an article by Weaver et al, entitled "A Study of Noninvasive Blood Pressure Measurement Technics", presented at a conference held at Stanford University in September 1978. This known system uses an algorithm for processing cuff pressure measurements and associated R-wave peak detector and Korotkov sound detector output signals for detecting artifact information in the output from the Korotkov sound detector and eliminating said artifacts from the blood pressure measurements.

Although the known system has been approved successfully the present invention involves an improved Korotkov sound processing system.

An object of this invention is the provision of an improved method of identifying false signals in the output from a Korotkov sound detector employed in apparatus for the measurement of blood pressure and/or systolic slope of blood pressure waves in an artery of a subject.

An object of this invention is the provision of an automatic computer-implemented technique for identifying and eliminating false outputs from a Korotkov sound detector included in a blood pressure measuring system, or the like, which technique is well adapted for use during stress testing, or in an operating room and intensive care units of hospitals where ECG signals normally are available.

The above and other objects and advantages of this invention are achieved by means of the method and apparatus as claimed in claims 1 and 9, respectively. The system includes an inflatable cuff which is inflatable to a pressure above systolic pressure and deflatable to a pressure below diastolic pressure. A pressure transducer connected to the inflatable cuff generates a signal which is a function of cuff pressure. A microphone picks up Korotkov sounds, and artifacts, during deflation of the cuff, and electrodes attached to the subject pick up electrocardiographic signals. The peak of the ECG R-wave is detected by R-wave peak detection techniques. Many, but not all artifacts, are removed from the microphone output by use of K-sound detection techniques.

The pressure transducer output is converted to digital form for transfer to a digital computer and storage in the computer memory. The time of arrival of the peak R-wave signals and associated K-sound and artifact signals also is stored in the computer memory. RK intervals or RK values comprising the time interval between the time of arrival of an R-wave signal and the associated K-sound or artifact signal are determined which, together with the associated cuff pressure, establish a plurality of RK values versus cuff pressure points, some of which are true points and others of which are artifact points. These points are stored in the computer memory. Certain RK values outside normal ranges are eliminated as probably resulting from artifacts. Remaining RK values are grouped in separate groups using a chaining operation which involves estimating from one point at a high cuff pressure, and RK value, $\hat{R}K$, at a next lower cuff pressure at which there is a point. If the difference between the RK value at said lower cuff pressure and estimated interval value, $\hat{R}K$, is within a selected range, the actual value is added to the group. Now, using the newly added point, an estimated RK value for the next lower cuff pressure at which there is another point, is calculated and the difference between the estimated value $\hat{R}K$ and actual RK value is determined. Again, the point is added to the group of points if the difference is within a predetermined range of RK values. If the difference is outside the range, the point is not added to the group of points, and the RK value—also called "RK interval" in the following description at the next lower cuff pressure containing a point is checked. After the point at the lowest cuff pressure has been checked, the process is repeated starting at the highest ungrouped point. When all of the points have been grouped, the group with the greatest number of RK values vs cuff pressure points therein is selected as the group which includes the greatest number of valid, or true, RK values versus cuff pressure points. A straight line using minimum mean-square fitting techniques is fitted to these points and, with further processing, some additional artifact points may be deleted from the group. With artifact points deleted, the cuff pressure at the maximum and minimum RK interval points provides a measure of the subject's systolic and diastolic blood pressures, respectively. Using the techniques of this invention, accurate blood pressure measurements may be obtained with ambulatory subjects in a noisy environment.

Brief description of the drawings

The invention will be better understood from the following description when considered with the accompanying drawings. In the drawings:

Fig. 1 shows peak R-wave detector, K-sound detector, and cuff pressure transducer output measurements obtained from a subject, plotted against time;

Fig. 2 is a plot of RK values versus cuff pressure with no artifacts present;

Fig. 3 is a plot of RK values versus cuff pressure which is similar to that of Fig. 2 but which results when artifacts are included in the output from the K-sound detector used in making the measurements;

Fig. 4 is a simplified block diagram of a system which may be employed in the practice of this invention for identifying and deleting K-sound artifacts from the output of K-sound detector means;

Fig. 5A and 5B, together, show a flow chart for use in explaining operation of this invention;

Fig. 6 is a plot of RK values versus cuff pressure showing points thereof joined in separate groups;

Fig. 7 is a flow chart showing details of a chaining operation included in steps of the flow chart of Figs. 5A and 5B, and

Fig. 8 is a plot of RK values versus cuff pressure for use in explaining the chaining operation depicted in Fig. 7.

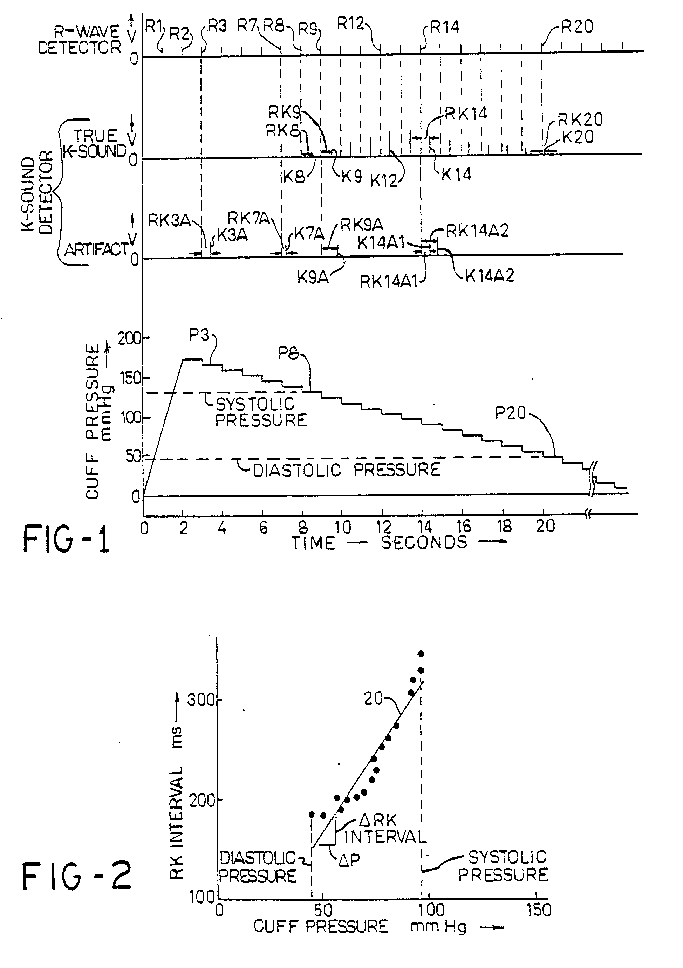

As is understood, electrocardiographic signals picked up by electrodes attached to a subject's body include a large R-wave component which is relatively easily detected. In Fig. 1, successive outputs from a peak R-wave detector included in the present system are identified by reference characters R1, R2 . . . R20. At any given level of physical activity substantially periodic peak R-wave signals are produced by the subject, and, for simplicity, periodically occuring R-wave signals are shown in Fig. 1. An inflatable cuff attached to the upper arm of the subject is inflated and slowly deflated as shown by the plot of cuff pressure versus time included in Fig. 1. The cuff is inflated to a pressure, P, above systolic pressure, then is deflated through a pressure range at which Korotkov sounds are produced. A microphone at the inflatable cuff senses Korotkov sounds during deflation of the cuff. The microphone output is coupled to a Korotkov sound detector, the output from which includes true K-sounds identified by reference characters K8 through K20. In Fig. 1 the numerical suffixes 1 through 20 for the R-wave detector output identify the successive heartbeat periods, with corresponding suffixes being used for the K-sound detector and pressure transducer outputs K and P, respectively.

In Fig. 1, the Korotkov sound detector output is depicted on two lines, the upper one of which includes only artifact-free K-sounds, and the lower one of which includes only artifacts. The letter suffix A identifies those K sound detector outputs which are artifacts, and, where more than one artifact output is produced during a given heartbeat period, a subsequent numerical suffix is included to separately identify the same. Thus, in Fig. 1, for heartbeat period 14, two K-sound artifact signals K14A1 and K14A2 are shown. For heart beat periods 3, 7 and 9, only one artifact per beat is shown, which are identified by reference characters K3A, K7A and K9A, respectively. In signal processing for the removal of artifacts from true K-sounds, use is made of the time interval between the occurrence of the R-wave and the subsequent occurrence of the associated K-sound detector output. Such RK intervals are identified by reference characters RK together with a numerical suffix for the associated heart beat periods. Where the K-sound detector output comprises an artifact, an additional suffix A is included, and if more than one artifact per heart beat period is produced, a subsequent numerical suffix distinguishes between the same. Thus, in Fig. 1, RK intervals for true Korotkov sounds are identified by reference characters RK8 through RK20 while RK intervals for artifacts are identified by the reference characters RK3A, RK7A, RK9A, RK14A1 and RK14A2.

Reference now is made to Fig. 2 of the drawings wherein a plot of RK interval measurements as a function of cuff pressure is shown. In Fig. 2, the plot includes RK interval measurements obtained using true K-sounds only, and not artifacts. Such RK interval versus cuff pressure points are identified as "true" points herein to distinguish the same from "artifact" points obtained using K-sound detector outputs which are artifacts. For the artifact-free plot shown in Fig. 2, a straight line 20 with a positive slope can be fitted through the series of points using minimum mean-squared fitting techniques. The slope, $\Delta$RK interval/$\Delta$ pressure, of the line 20, is inversely proportional to the systolic slope of the blood pressure wave of the subject and, therefor, provides a measure of the systolic slope of the blood pressure wave. The systolic slope of the blood pressure wave, and hence the slope of line 20, varies in accordance with exercise, with the systolic slope generally slowly increasing with increasing exercise. At rest, before exercise, a slope of approximately 1 (one) for line 20 is typical. In the operation of this invention a "control slope" $m_c$ is computed using an estimated slope of, say, 1 for the line 20 while the subject is at rest, before exercise. Measurements of slope are described in detail below. Also, the maximum and minimum cuff pressures at which true Korotkov sounds are obtained provide a measure of the systolic and diastolic blood pressures, respectively, as seen in Fig. 2.

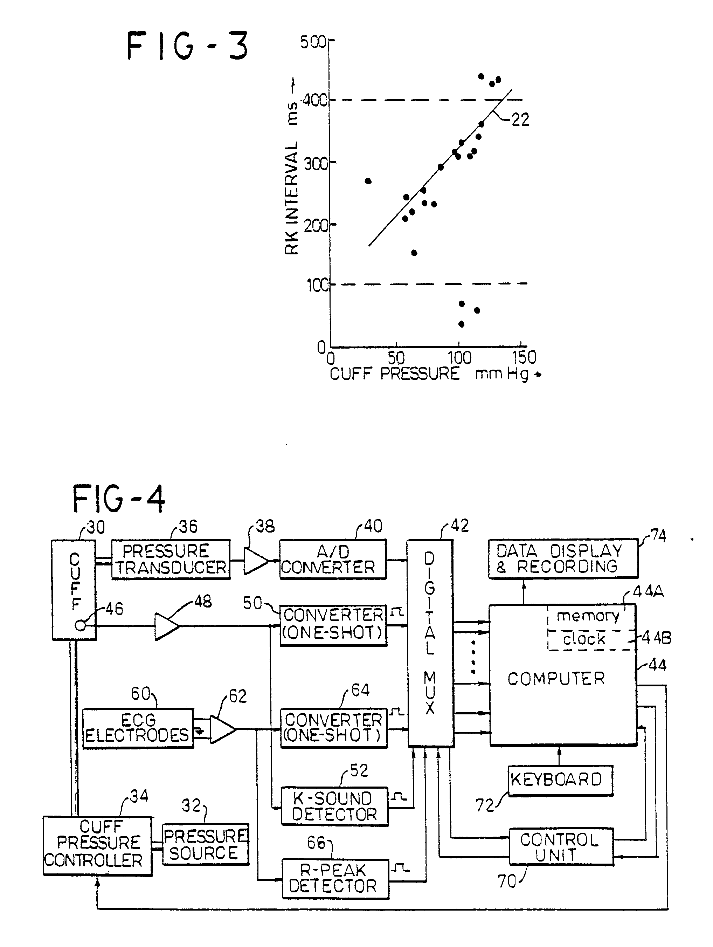

For an ambulatory subject, or one undergoing stress exercise, the K-sound detector output includes numerous artifacts. In Fig. 3, to which reference now is made, a plot of RK interval measurements as a function of cuff pressure is shown which includes not only true points but also includes artifact points. A straight line 22 is shown fitted to the points using minimum means-squared fitting techniques. Although the slope of the line 22 may approximate that of a curve fitted to only true points, artifact points must be substantially eliminated from the plot if a relatively true measure of systolic slope and/or of systolic or diastolic blood pressure is to be obtained from the detected R-waves and K-sounds and associated cuff pressure measurements. An algorithm using cuff pressure measurements and times of occurrence of R-wave and K-sound outputs for identifying artifacts in order that they may be deleted from the K-sound output is described below. First, however, a simplified showing of a system which may be used for implementing such an algorithm will be described, which system includes means for obtaining necessary measurments of cuff pressure along with measurements of the time of arrival of R-waves and K-sounds.

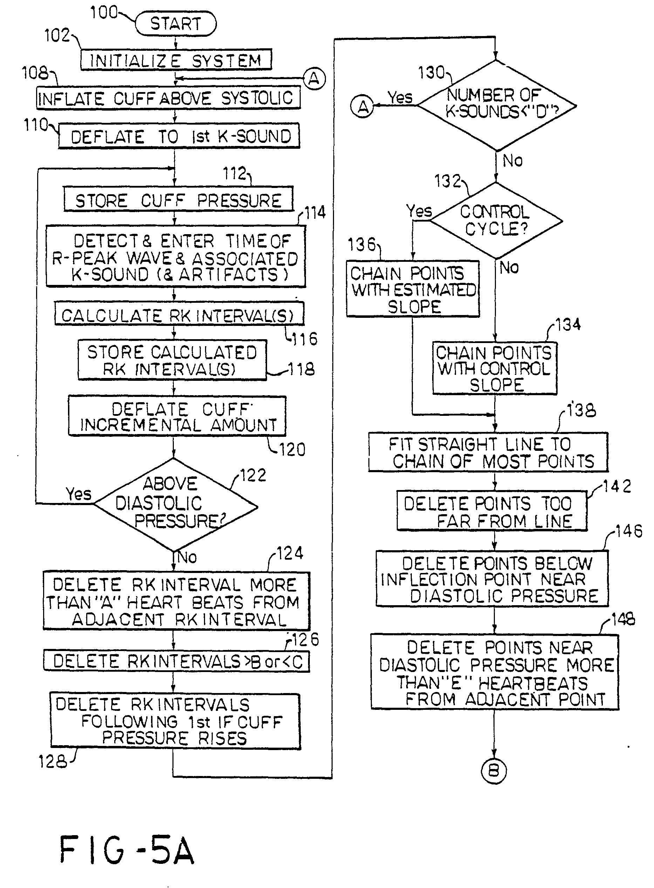

Reference now is made to Fig. 4 wherein a system which is suitable for use in the practice of this invention is shown comprising an inflatable cuff 30 for encircling a subject's limb, such as upper arm, and a pressure source 32 connected to the cuff through a pressure controller 34. Cuff pressure is sensed by a pressure transducer 36, the analog output from which is connected through an amplifier 38 to the input of an analog to digital converter 40 for conversion to digital signal form. The digitized cuff pressure signal is connected through a digital multiplexer 42 to a computer 44 which includes memory 44A where cuff pressure signals obtained during a cuff deflation temporarily are stored for use in computing systolic and diastolic blood pressure and/or the systolic slope of the blood pressure waves during said cuff deflation.

With the cuff attached to the upper arm of the subject, the cuff is inflated to a pressure above systolic pressure. Then, as the cuff pressure is decreased, the first korotkov sound appears at the systolic pressure, and the last at the diastolic pressure. A microphone 46 picks up the Korotkov sound (K-sound) at a plurality of cuff pressures between systolic and diastolic pressures. The microphone also picks up noise; the amount of noise produced depending upon the physical activity of the subject. The microphone output signal is amplified by amplifier 48, and the amplifier output is supplied both to a signal converter 50 and to a K-sound detector 52. The converter 50 simply may include a one-shot for generation of a pulse output in response to an amplified output signal from amplifier 48, which pulse output is connected to the multiplexer 42. The K-sound detector 52 distinguishes between true K-sounds and some artifacts, and produces an output in response to true K-sounds and artifacts which are not eliminated by the detector. The K-sound detector output is connected to an address input of the multiplexer 42. In the presence of an output from the K-sound detector, the output from converter 50 is connected through the multiplexer 42 to an interrupt input of the computer 44 to produce a K-sound, or artifact, timing signal which, together with an associated R-wave timing signal, provides a measure of the RK interval.

ECG electrodes 60 attached to the subject's body pick up ECG signals which are amplified by amplifier 62 and then supplied to a converter 64 and to an R-peak detector 66. As with the converter 50, the converter 64 also may include a one-shot for generation of a pulse output in response to the R-wave component of the amplified ECG signal. The pulse output from the converter 64 is connected to the multiplexer 42 for connection as an interrupt input to the computer 44. The R-peak detector detects the R-wave of the ECG signal while discriminating against noise and other ECG signal components, such as the P and T wave components. The R-peak detector output is supplied as an address input to the multiplexer 42 for connection of the output from the converter 64 to an interrupt input of the computer 44 when an R wave is detected. The difference in time between the arrival of an R wave input and associated true and/or artifact K-sound signal(s) at the interrupt inputs to the computer provides a measure of the RK and/or RKA interval, which interval value is temporarily stored in the computer memory 44A for use with other such true and artifact K-sound interval values obtained at different cuff pressures. A clock 44B is included in the system for use in making the above-described time interval measurements.

Another address input for the multiplexer 42 is obtained from the computer 44 through a control unit 70. Under control of unit 70, the multiplexer 42 is switched for connection of cuff pressure signals from the A/D converter 40 to the computer 44. Also, multiplexer address information is supplied to the computer 44 through the control unit 70 for use by the computer in controlling operation of the multiplexer. A keyboard 72 may be included for manual supply of information to the computer, such as the name of the subject being tested, etc. Data display and recording unit 74 may be used to display and/or record information output from the computer, such as systolic slope, systolic and diastolic blood pressure, or the like.

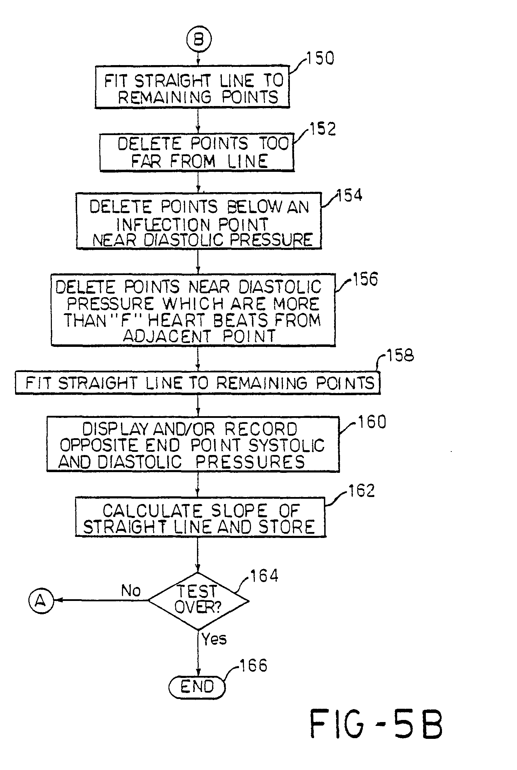

The computer 44 implements a novel process for identifying, and eliminating, artifacts included with true K-sound signals in order that accurate blood pressure and/or systolic slope measurements may be obtained using only true K-sounds. The process, in general, will be best understood with reference to the flow chart of Figs. 5A and 5B. It will here be noted that one or more programming steps may be involved in the actual implementation of the indicated operation. Since the programming of such steps for the indicated operations is well within the skill of the average programmer, a complete program listing is not required and is not included herein.

With the cuff 30 and transducers 36 and 60 properly secured to the subject, the test is started as indicated by START step 100, at which time system power is turned on or a reset operation is performed, by means not shown. Initialization step 102 includes initial settings of counters, registers and the like, in the computer 44. A control slope $m_c$ is used by the algorithm for identifying, and discarding, artifacts. For the first activity, R-peak, K-sound and cuff pressure measurements are obtained while the subject is at rest. Measurements obtained during this resting activity are used for computing an initial control slope $m_c$. As noted above, the K-sound detector output is relatively free of artifacts while the subject is at rest, thereby ensuring that a relatively accurate value of control slope $m_c$ is computed.

With the subject at rest, before exercise, cuff inflation step 108 is entered wherein the cuff 30 is inflated under control of the computer to a pressure above systolic blood pressure through operation of the cuff pressure controller 34 to occlude blood flow in the brachial artery. Next, at step 110, the cuff pressure is reduced to a pressure at which true Korotkov, or artifact, sounds are first detected which, for true Korotkov sounds, is the systolic blood pressure. At this point, the cuff pressure is entered into the computer memory 44A through use of the transducer 36, amplifier 38, A/D converter 40 and digital multiplexer 42, as indicated by step 112.

Next, at step 114, and R-peak wave is detected and its time of arrival is entered into the computer memory. The time of arrival of an associated Korotkov sound also is entered into the computer memory, as is the time of arrival of artifacts, if any, in the K-sound detector output. As seen in Fig. 1 and described above, for any given R-peak wave, the time of arrival of the true K-sound and that of one or more artifacts may be stored.

At step 116 the RK interval is calculated, and the RK interval value, or values, are stored (step 118) with the associated cuff pressure. The cuff pressure, at step 120, is then reduced an incremental amount of, say, 4 mmHg (530 Pa). The decision step 122 next is performed to determine whether or not cuff pressure remains above diastolic pressure. If the decision is affirmative, step 112 is again entered, whereupon the new reduced cuff pressure value is stored, together with new associated RK interval values.

When the cuff pressure is reduced below diastolic pressure, decision step 122 is negative, and step 124 is entered and the process of eliminating artifacts from the K-sound detector output begins. At step 124, RK intervals calculated at step 116 are checked for the presence of an interval which is more than a predetermined number, A, of heartbeats from an adjacent RK interval. Any RK interval which is more than, say, 3 heartbeats from an adjacent interval is deleted from the list of RK intervals stored at step 118. In Fig. 1, RK interval RK3A (occurring at heartbeat period 3) is more than three heartbeats from the nearest RK interval (here, RK7A at heartbeat period R7) and, in accordance with step 124 is deleted from the store of RK intervals obtained during the illustrated cuff deflation. Generally, such isolated RK intervals result from artifacts and should not be included in subsequent computations.

For any subject under any exercise condition, RK intervals are practically never less than 100 ms and rarely are greater than 400 ms. At step 126, the store of RK intervals is checked and those intervals equal to or less than B, say, 100 ms or equal to or greater than C, say, 400 ms, are deleted from the list. Minimum values employed in this test may range, for example, from 25 to 150 ms, and maximum values from 350 to 600 ms.

Under certain conditions during a cuff deflation, the cuff pressure may rise, due, for example, to physical pressure exerted thereon during exercise. At step 128, the cuff pressure is checked for any rise therein which may have occured during a cuff deflation. If there has been a pressure rise during a heartbeat period, only the first-occuring RK interval is accepted, and any subsequent RK intervals which may occur during the period are deleted. K-sound detector outputs which occur during the pressure increase often are produced by sounds generated by such increase and are assumed to be artifacts.

At decision step 130, the total number of RK intervals remaining following steps 124 through 128 is checked. If the number is less than, D, the RK intervals for the deflation are not processed, and operation returns to step 108 for the start of another cycle of operation. With 4 mmHg (530 Pa) steps during cuff deflation, a plurality of true K-sounds normally are produced. For this test, a value of D in the range of between 4 and 10 typically is employed. It will be apparent that steps 124 through 128 may be performed in any desired order.

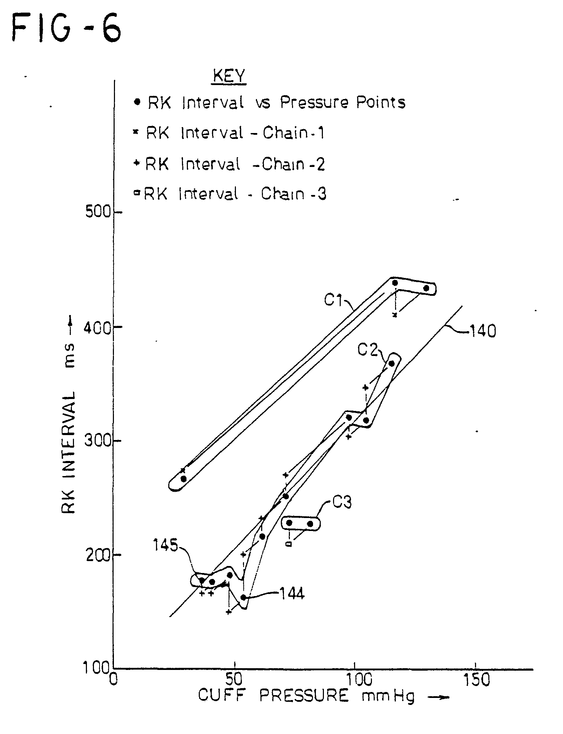

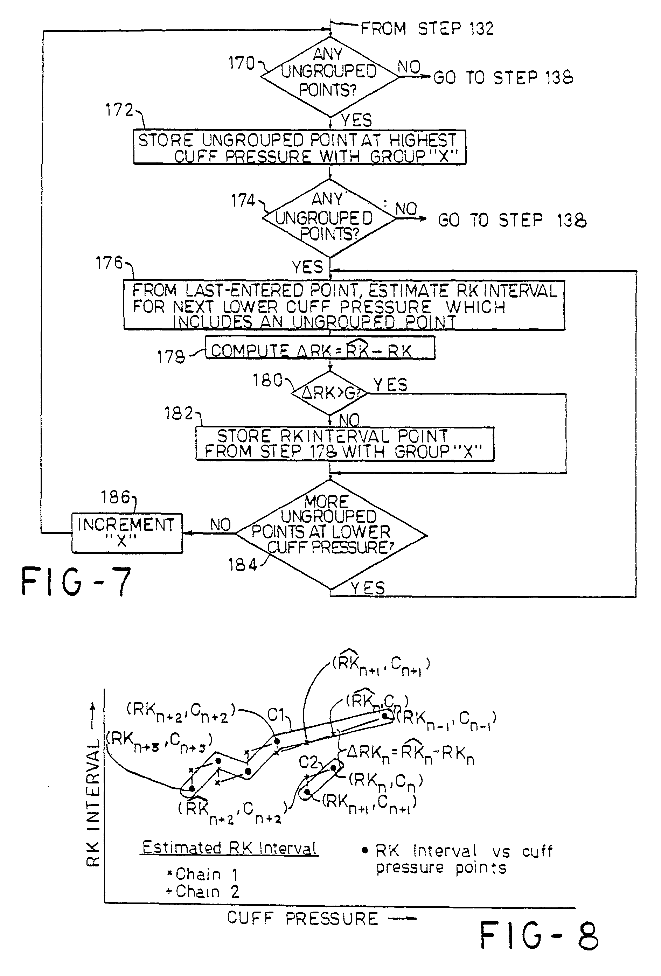

If a sufficient number of RK interval points remain after step 130, another decision step 132 is entered at which a determination is made whether or not a control cycle is being processed. As noted above, once a control slope $m_c$ has been determined, such a control slope, or some function thereof, may be used in subsequent chaining operations. Chaining involves grouping together RK interval points, in a manner described below, to eliminate those points which fail to fit the group which includes the most points. If a control slope has been determined during a prior cycle, or cycles, of operation, such control slope is used in the chaining operation which follows, as indicated at step 134. If no such control slope has been established, an estimated slope is used for the chaining operation, as indicated at step 136. In Fig. 6, a plot of RK interval versus cuff pressure is shown wherein three groups, or chains, of RK interval points have been established using the chaining process, the groups being identified by reference characters C1 through C3. Details involved in the chaining steps 134 and 136 are included in the flow chart of Fig. 7, described below following the completion of the description of the flow chart of Figs. 5A and 5B.

The chain which includes the greatest number of RK interval points established at step 134, or at step 136, is selected for the next step 138 where a straight line is fitted to the chain using a minimum mean-squared algorithm. In Fig. 6, straight line 140 is shown fitted to chain C2 in accordance with step 138 of the flow chart. At step 142, the distance of the RK interval points in the chain from the straight line 140 is determined, and all points more than a specified distance therefrom are deleted from the chain. For example, RK interval points more than, say, 20 ms from the line 140 may be deleted. In Fig. 6, RK interval point identified by reference character 144 is eliminated by operation of step 142.

At step 146, a check of RK interval points adjacent the diastolic end of the chain is made to determine whether or not the RK interval of the end point is less than that of its neighboring point. An end point (such as point 145 shown in Fig. 6) having an RK interval greater than the neighboring point is deleted from the chain. When an end point is deleted, the above test is repeated until an end point having an RK interval which is less than the neighboring point is located. In this manner, a point in the chain adjacent diastolic pressure at which there is an upward deflection of the chain is selected as an end point.

Next, at step 148, another check of points adjacent the diastolic end of the chain is made for any point, or group of, say, two points which is more than E heartbeats from an adjacent point, where E is, say, 3. Such point, or group of points, is deleted from the chain as likely being artifacts.

At the following step 150, a straight line is fitted to the remaining points of the selected chain using the minimum mean-squared algorithm in the manner of step 138. Steps 152, 154 and 156 which follow are similar to the above-described steps 142, 146 and 148 respectively, except the operations now are performed on the chain of RK interval points and straight line fitted thereto at step 150. At step 158, another straight line is fitted to the points which remain. The cuff pressures at the shortest and longest RK intervals included in the chain are a measure of the respective diastolic and systolic blood pressure of the subject, which pressure measurements may be displayed, stored, recorded or the like, as indicated at step 160. The slope of the straight line fitted at step 158 is determined at step 162, which slope provides a measure of the systolic slope of the blood pressure waves during the cuff deflation. This value may be stored, recorded, displayed, or the like, as desired. At decision step 164, step 108 is reentered if the rest is to be continued. If not, the test is ended at step 166.

Reference now is made to the flow chart of Fig. 7 which includes details of the chaining operation shown at step 134 of Fig. 6A. As noted above, chaining involves the dividing of RK interval versus cuff pressure points obtained during a cuff deflation into groups. At step 170, a check is made for any ungrouped points. Of any remaining points, that point at the highest cuff pressure is stored in a first group of points at step 172. Referring to the RK interval versus cuff pressure plot of Fig. 8, at the beginning of the chaining operation, point $(RK_{n-1}, C_{n-1})$, which is the highest ungrouped cuff pressure point, is stored in group, or chain, 1 (one) of points at this step. Next, at step 174, another check is made for any ungrouped points and, if any points remain, an estimate of the RK interval for the next lower cuff pressure point is made at step 176. In Fig. 8, the point at the next lower cuff pressure is point $(RK_n, C_n)$. An estimated RK interval $\hat{RK}_n$ for cuff pressure $C_n$ is computed by projecting a line with a control slope $m_c$ from point $(RK_{n-1}, C_{n-1})$ to $C_n$. The difference between the estimated and actual RK values is determined at step 178. In Fig. 8, this difference is identified as

$$\Delta RK_n = \hat{RK}_n - RK_n.$$

If an artifact is present, the absolute difference $\Delta RK_n$ almost always falls outside the 40 ms, range, and at step 180 $\Delta RK_n$ is compared to, say, 40 ms to determine whether or not it is within the 40 ms range. If it is within this range, the point $(RK_n, C_n)$ is stored in a group with point $(RK_{n-1}, C_{n-1})$ at step 182, and decision step 184 is entered. If not within range, decision step 184 is entered without storing, or grouping the point. For the plot of Fig. 8, point $(RK_n, C_n)$ is outside the range whereby point $(RK_n, C_n)$ is not stored with the group 1 points.

At step 184, a check for additional points at lower cuff pressures is made. If it is determined that one or more such points remain, step 176 is reentered, wherein an estimated RK interval for the next lower cuff pressure point is made. In Fig. 8, the next lower cuff pressure point is $(RK_{n+1}, C_{n+1})$, and the estimated point is $(\hat{RK}_{n+1}, C_{n+1})$. Again, the difference in RK value between the actual and estimated points exceeds 40 ms, whereby this point also is not included with the group 1 points.

The next lower cuff pressure point $(RK_{n+2}, C_{n+2})$ is checked and the $\Delta RK_n$ is determined to be within the 40 ms range. Now, the result of decision step 180 is negative, whereupon step 182 is entered for storage of point $(RK_{n+2}, C_{n+2})$ with the group 1 points.

In accordance with the present invention, when a new RK value vs cuff pressure point is added to the group, a line with the control slope $m_c$ is projected from the newly added point to the next lower cuff pressure which includes an ungrouped point. As seen in Fig. 8, a new line with slope $m_c$ is shown projected from point $(RK_{n+2}, C_{n+2})$ newly added to the list. This method of chaining provides for much more accurate grouping of points than is provided in the arrangement disclosed in the above mentioned article, "A Study of Noninvasive Blood Pressure Measurement Techniques" wherein the line is projected from the highest cuff pressure points to the lowest, and not from newly added points.

In Fig. 8, it will be seen that $(RK_{n+5}, C_{n+5})$ is the last point to be added to chain 1. At this point in the operation, decision step 184 is negative, whereupon the group number is incremented at step 186, and decision step 170 is reentered. If more ungrouped points exist, the chaining operation is repeated starting with the point at the highest cuff pressure not yet included in a chain, or group. In Fig. 8, point $(RK_n, C_n)$ is selected at step 172 for storage with points of a second group. After point $(RK_{n+1}, C_{n+1})$ has been identified as a group 2 point, at step 180, and stored at step 182, no more ungrouped points at a lower cuff pressure remain, and the operation loops back through step 186 to step 170. Now, since there are no remaining ungrouped points, decision step 170 is negative, and step 138 (Fig. 5A) is entered for fitting of a straight line to the group which includes the greatest number of points in the manner described above.

Step 136 involves essentially the same operations as above-described step 134 except that an estimated slope rather than a control slope is used in projecting a line from one point to a next lower cuff pressure which includes a point. As noted above, the estimated slope is used during a control cycle during which the subject is stationary, and artifacts are at a minimum. The slope of the straight line fitted to points at step 158 and

calculated at step 162 during a control cycle is employed in step 134 on subsequent cuff deflations during which the subject is active. If desired, a plurality of control cycles may be performed, and the average slope obtained therefrom may be used as the control slope.

The slope of the line fitted at step 158 normally changes during exercise. For any given cuff deflation, the slope obtained during one or more preceding cuff deflations, or some function thereof, may be used in step 134 as the control slope. The cuff deflation cycles may be repeated often enough such that the change in slope between cycles is minimal. Thus, the slope obtained during one cuff deflation may provide an accurate control slope for use in a next cycle. In an alternative arrangement, an estimated slope of, say, 1 may be employed in all chaining operations, without establishment of a control slope.

The invention having been described in detail various other changes and modifications will suggest themselves to those skilled in this art. For example, the novel method is not limited to use with apparatus illustrated in Fig. 4. Instead of using R-wave and K-sound detectors, the ECG signal and/or Korotkov sound waveforms may be digitized, and the digital signals supplied to the computer for software R-wave and/or K-sound detection, with the time of arrival of the software R-wave and/or K-sounds being stored in the computer memory. Also, a recording of the necessary inputs may be made, and the recorded signals played back to provide the computer inputs. Such recording of signals for processing is particularly useful for long term monitoring of blood pressure of ambulatory subjects. Portable equipment for automatic cuff inflation and deflation, and K-sound, ECG and cuff pressure recording is well known. Obviously, high speed playback of the recorded signals is possible, so long as compensation is made for any time differences, if any, which may result therefrom.

**Claims**

1. Method for identifying and removing artifacts from Korotkov sound signals comprising the steps of:

1) repeatedly obtaining cuff pressure measurements and arrival times of peak R-wave signals and Korotkov sound signals including artifacts through the active K-sound range of a cuff pressure deflation,

2) using the arrival times of the R-wave signals and associated Korotkov sound signals and artifacts to determine RK values, defined as the intervals between the arrival times of R-wave signals, and the associated Korotkov sound signals and artifacts, from which RK values a plot of RK values versus cuff pressure points is obtained, in which plot some are true points, produced by the Korotkov sound signals, and others are artifact points,

3) processing said points to group the same such that one group includes substantially only true points, by excluding points whereof the differences $\Delta RK = \hat{RK} - RK$ is outside a predetermined range, wherein $\hat{RK}$ is an estimated RK value of the point on the plot at the next lower cuff pressure at which there is a point and RK is the actual value at said point by using the projection of a straight line having a predetermined slope m from a RK value versus cuff pressure point in order to chain the points, and using the following method steps:

a) estimating a RK value, $\hat{RK}$, by starting from the point at the highest cuff pressure, for the next lower cuff pressure which includes a point and using the line of slope m,

b) including the point at said lower cuff pressure in a group which includes also said point at the highest cuff pressure if the difference $\Delta RK = \hat{RK} - RK$ is below a given value, otherwise excluding the point at said lower cuff pressure from that group and

c) repeating steps 3a) and 3b) until all points are processed, characterized by the following method steps:

d) repeating steps 3a) and 3b) using the last included point in the group for estimating the RK value for the next lower cuff pressure which includes a point, using the straight line of the predetermined slope m from said last included point in the group,

e) repeating steps 3a), 3b) and 3c) until all points are included in one or other group, and

4) selecting as the one group which includes substantially only true points that group which includes the greatest number of points.

2. Method as claimed in claim 1, characterized by

5) obtaining a measure of systolic and diastolic blood pressures from points adjacent the respective upper and lower ends of cuff pressures for the selecting step 4).

3. Method as claimed in claim 1 or 2, characterized by

6) fitting a straight line to at least some points of the selected one group of substantially only true points from step 4), the slope of said straight line providing a measure of the systolic slope of the blood pressure waves during cuff deflation.

4. Method as claimed in one of the preceding claims, characterized in that the predetermined slope m is an estimated slope.

5. Method as claimed in claim 4, characterized in that an estimated slope m of substantially 1 is employed (wherein the RK value is in units of ms and cuff pressure is in units of mmHg).

6. Method as claimed in one of the claims 1—3, characterized in that the predetermined slope m is established during a previous operating cycle.

7. Method as claimed in one of the preceding claims, characterized by deleting from said one group any points adjacent diastolic pressure having RK values larger than the smallest RK value at cuff pressures less than said cuff pressure at said smallest interval.

8. Method as claimed in one of the preceding claims, characterized by using minimum mean-squared fitting techniques, fitting a straight line to said one group of points, and repeating steps 3a) through 3c) using the slope of the straight line to obtain the estimated value $\hat{RK}$.

9. System for identifying and removing artifacts in Korotkov sound signals, comprising:

1) obtaining means (36, 48) for repeatedly obtaining cuff pressure measurements and arrival times of peak R-wave signals (R) and Korotkov sound signals (K) including artifact sound signals (KA) through the active K-sound range of the deflation of a cuff (30),

2) detectors (52, 66) for determining a plot of RK values versus cuff pressure points, some of which are true points and others of which are artifact points,

3) processing means (44) for processing said points to group the same such that one group includes substantially only true points, and to exclude artifact points including

a) estimating means responsive to one point at the highest cuff pressure for estimating an RK value ($\hat{RK}$) for a true point at a next lower cuff pressure which includes a point,

b) combining means for including said point at said next lower cuff pressure in a group which includes said one point if the difference ($\Delta RK$) between the estimated RK value ($\hat{RK}$) and the RK value (RK) at said next lower cuff pressure is below a given value, otherwise excluding said point from the group,

c) repeating means for repeating operations performed by elements 3a) and 3b) until no ungrouped point at a lower cuff pressure remains,

d) further repeating means for repeating operations performed by elements 3a), 3b), and 3c) until all points are included in a group, points identified by element 3d) being included in another group of points each repetition of element 3d) operations, wherein the estimating means and/or processing means (44) are responsive to a line of slope (m) projected from said one point,

characterized in that the repeating means includes using the last-added point to the group for estimating RK values ($\hat{RK}$) and that

4) identifying means identify the group with the largest number of points as a group of substantially all true points.

10. System as claimed in claim 9, characterized in that an estimated slope (m) is stored in the processing means (44).

11. System as claimed in claim 9, characterized in that the slope (m) comprises a control slope obtained from a prior cycle of operation.

12. System as claimed in one of the claims 9—11, characterized in that the obtaining means (36, 48) obtain a measure of systolic and diastolic blood pressures from points adjacent the upper and lower ends, respectively, of cuff pressures of the group of substantially all true points.

13. A system as claimed in one of the claims 9—12, characterized in that fitting means fit a straight line to at least some of the points of the group of substantially all true points, the slope (m) of said straight line comprising a measure of the systolic slope of blood pressure waves during cuff deflation.

**Patentansprüche**

1. Verfahren zum Erkennen und Entfernen von Artefakten aus Korotkow-Tonsignalen, umfassend folgende Schritte:

1) wiederholtes Ableiten von Manschettendruckmessungen und Ankunftszeiten von R-Zakkensignalen und Korotkow-Tonsignalen einschließlich Artefakten durch den aktiven K-Tonbereich bei sinkendem Manschettendruck,

2) Nutzen der Ankunftszeiten der R-Zackensignale und zugehörigen Korotkow-Tonsignale und Artefakte zur Bestimmung von RK-Werten, die definiert sind als die Intervalle zwischen den Ankunftszeiten von R-Zackensignalen und den zugehörigen Korotkow-Tonsignalen und Artefakten, wobei aus diesen RK-Werten eine Aufzeichnung von RK-Werten gegenüber Manschettendruckpunkten erhalten wird und in dieser Aufzeichnung einige Punkte echte Punkte sind, die durch die Korotkow-Tonsignale gebildet sind, und andere Artefakt-Punkte sind,

3) Verarbeiten dieser Punkte unter Gruppierung derselben derart, daß eine Gruppe im wesentlichen nur echte Punkte umfaßt, indem Punkte ausgechlossen werden, bei denen die Differenzen

$$\Delta RK = \hat{RK} - RK$$

außerhalb eines vorbestimmten Bereichs liegen, wobei $\hat{RK}$ ein geschätzter RK-Wert des Punkts auf der Aufzeichnung beim nächstniedrigeren Manschettendruck ist, bei dem ein Punkt vorhanden ist und RK der Ist-Wert an diesem Punkt ist, indem die Projektion einer Geraden mit einer vorbestimmten Neigung m von einem RK-Wert/Manschettendruckpunkt genützt wird zum Verketten der Punkte, und wobei folgende Verfahrensschritte angewandt werden:

a) Schätzen eines RK-Werts $\hat{RK}$, indem von dem Punkt beim höchsten Manschettendruck ausgegangen wird, für den nächstniedrigeren Manschettendruck, der einen Punkt einschließt, und Nutzen der Geraden mit der Neigung m,

b) Einschließen des Punkts bei dem genannten niedrigeren Manschettendruck in eine Gruppe, die auch den genannten Punkt beim höchsten Manschettendruck einschließt, wenn die Differenz

$$\Delta RK = \hat{RK} - RK$$

unter einem gegebenen Wert liegt, und andernfalls Ausschließen des Punkts bei dem niedrigeren Manschettendruck von dieser Gruppe, und

c) Wiederholen der Schritte 3a) und 3b), bis sämtliche Punkte abgearbeitet sind, gekennzeichnet durch die folgenden Verfahrensschritte:

d) Wiederholen der Schritte 3a) und 3b) unter Nutzung des letzten eingeschlossenen Punkts in der Gruppe zum Schätzen des RK-Werts für den nächstniedrigeren Manschettdruck, der einen Punkt einschließt, unter Nutzung der Geraden mit der vorbestimmten Neigung m ausgehend von dem zuletzt eingeschlossenen Punkt der Gruppe,

e) Wiederholen der Schritte 3a), 3b) und 3c), bis sämtliche Punkte in der einen oder anderen Gruppe eingeschlossen sind, und

4) Auswählen derjenigen Gruppe, die die größte Anzahl Punkte enthält, als die eine Gruppe, die im wesentlichen nur echte Punkte umfaßt.

2. Verfahren nach Anspruch 1, gekennzeichnet durch

5) Ableiten eines meßwerts für den systolischen und den diastolischen Blutdruck aus Punkten, die an das jeweilige obere und untere Ende der Manschettendrücke angrenzen, für den Auswahlschritt 4).

3. Verfahren nach Anspruch 1 oder 2, gekennzeichnet durch

6) Ansetzen einer Geraden an wenigstens einige Punkte der ausgewählten einen Gruppe von im wesentlichen nur echten Punkten aus Schritt 4), wobei die Neigung dieser Geraden ein Maß für den systolischen Verlauf der Blutdruckwellen bei sinkendem Manschettendruck liefert.

4. Verfahren nach einem der vorhergehenden Ansprüche, dadurch gekennzeichnet, daß die vorbestimmte Neigung m eine geschätzte Neigung ist.

5. Verfahren nach Anspruch 4, dadurch gekennzeichnet, daß eine geschätzte Neigung m von im wesentlichen Eins verwendet wird (wobei der RK-Wert in ms und der Manschettendruck in mmHg angegeben ist).

6. Verfahren nach einem der Ansprüche 1—3, dadurch gekennzeichnet, daß die vorbestimmte Neigung m während eines vorhergehenden Betriebszyklus erstellt wird.

7. Verfahren nach einem der vorhergehenden Ansprüche, gekennzeichnet durch Löschen aus der einen Gruppe aller an den diastolischen Druck angrenzenden Punkte, deren RK-Werte größer sind als der kleinste RK-Wert bei Manschettendrücken, die niedriger als der Manschettendruck bei dem kleinsten Intervall sind.

8. Verfahren nach einem der vorhergehenden Ansprüche, gekennzeichnet durch Ansetzen einer Geraden an die eine Gruppe von Punkten unter Anwendung von minimalen mittleren quadratischen Ansetzmethoden, und Wiederholen der Schritte 3a) bis 3c) unter Nutzung der Neigung der Geraden zum Erhalt des geschätzten Werts $\hat{RK}$.

9. Einrichtung zum Erkennen und Entfernen von Artefakten in Korotkow-Tonsignalen, umfassend:

1) Ableitelement (36, 48) zum wiederholten Erhalt von Manschettendruckmessungen und Ankunftszeiten von R-Zackensignalen (R) und Artefakt-Tonsignale (KA) enthaltenden Korotkow-Tonsignalen (K) durch den aktiven K-Tonbereich bei sinkendem Druck einer Manschette (30),

2) Detektoren (52, 66), die eine Aufzeichnung von RK-Werten gegenüber Manschettendruckpunkten bestimmen, von denen einige echte Punkte und andere Artefakt-Punkte sind,

3) einen Rechner (44) zum Verarbeiten dieser Punkte unter Gruppierung derselben derart, daß eine Gruppe im wesentlichen nur echte Punkte einschließt, und Ausschluß von Artefakt-Punkten, umfassend:

a) eine Schätzeinheit, die aufgrund eines Punkts beim höchsten Manschettendruck einen RK-Wert ($\hat{RK}$) für einen echten Punkt bei einem nächstniedrigeren Manschettendruck, der einen Punkt einschließt, schätzt,

b) eine Verknüpfungseinheit, die den Punkt bei dem nächstniedrigeren Manschettendruck in eine Gruppe einschließt, die den einen Punkt enthält, wenn die Differenz ($\Delta RK$) zwischen dem geschätzten RK-Wert ($\hat{RK}$) und dem RK-Wert (RK) bei dem nächstniedrigeren Manschettendruck unter einem vorgegebenen Wer liegt, und andernfalls diesen Punkt aus der Gruppe ausschließt,

c) Wiederholungsmittel zur Wiederholung von durch die Elemente 3a) und 3b) durchgeführten Operationen, bis kein nichtgruppierter Punkt bei einem niedrigeren Manschettendruck übrigbleibt,

d) weitere Wiederholungsmittel zur Wiederholung von durch die Element 3a), 3b) und 3c) durchgeführten Operationen, bis sämtliche Punkte in einer Gruppe eingeschlossen sind, wobei durch Element 3d) erkannte Punkte bei jeder Wiederholung von Element-3d)-Operationen in einer weiteren Gruppe von Punkten eingeschlossen werden, wobei die Schätzeinheit und/ oder der Rechner (44) auf eine von dem einen Punkt projizierte Gerade mit einer Neigung (m) ansprechen, dadurch gekennzeichnet, daß die Wiederholungsmittel die Nutzung des zuletzt der Gruppe zugefügten Punkts zum Schätzen von RK-Werten ($\hat{RK}$) umfassen und daß

4) eine Erkennungseinheit die Gruppe mit der größten Anzahl Punkte als eine Gruppe mit im wesentlichen nur echten Punkten erkennt.

10. Einrichtung nach Anspruch 9, dadurch gekennzeichnet, daß in dem Rechner (44) eine geschätze Neigung (m) gespeichert ist.

11. Einrichtung nach Anspruch 9, dadurch gekennzeichnet, daß die Neigung (m) eine aus einem früheren Operationszyklus erhaltene Kontrollneigung umfaßt.

12. Einrichtung nach einem der Ansprüche 9—11, dadurch gekennzeichnet, daß die Ableitungselement (36, 48) ein Maß für die systolischen und diastolischen Blutdrücke aus Punkten ableiten, die an das obere bzw. das untere Ende von Manschettendrücken der Gruppe von im wesentlichen nur echten Punkten angrenzen.

13. Einrichtung nach einem der Ansprüche 9—12, dadurch gekennzeichnet, daß Ansetzmittel eine Gerade an wenigstens einige der Punkte der Gruppe von im wesentlichen nur echten Punkten ansetzen, wobei die Neigung (m) der genannten Geraden ein Maß für den systolischen Verlauf von Blutdruckwellen bei sinkendem Manschettendruck umfaßt.

## Revendications

1. Procédé pour identifier et éliminer les artefacts des signaux de bruits de Korotkov comprenant les stades suivants:

1) obtention répétée de mesures de la pression de brassard et des temps d'arrivée de signaux d'ondes R de pointe et de signaux de bruits de Korotkov comprenant des artefacts parmi les bruits K actifs d'un abaissement de la pression de brassard,

2) utilisation des temps d'arrivée des signaux d'ondes R de pointe et des signaux associés de bruits de Korotkov et d'artefacts pour déterminer des valeurs RK définies comme les intervalles entre les temps d'arrivée de signaux d'onde R et les signaux de bruits de Korotkov et d'artefacts d'après lesquels on obtient un tracé de valeurs RK en fonction de points de la pression de brassard, certains de ces points du tracé étant des points véritables produits par les signaux de bruits de Korotkov tandis que d'autres sont des points artefacts,

3) traitement desdits points par les grouper de telle façon qu'un groupe ne comprenne essentiellement que des points véritables en excluant les points dont les différences

$$\Delta RK = \hat{RK} - RK$$

sont en dehors d'un intervalle prédéterminé, RK étant une valeur RK estimée du point du tracé à la pression de brassard inférieure suivante à laquelle il y a un point et RK étant la valeur réelle audit point par utilisation de la projection d'une ligne droite ayant une pente prédéterminée m à partir d'une valeur RK de pression de brassard afin d'enchaîner les points et par utilisation des phases de méthode suivantes:

a) estimation d'une valeur $\hat{RK}$ de RK en partant du point à la pression de brassard la plus élevée pour la pression de brassard inférieure suivant qui comprend un point et en utilisant la ligne de pente m,

b) inclusion du point à la dite pression de brassard inférieure dans un groupe qui comprend aussi ledit point à la pression de brassard la plus haute si la différence

$$\Delta RK = \hat{RK} - RK$$

est au-dessous d'une valeur donnée, en excluant d'autre part le point à ladite pression de brassard inférieure de ce groupe et

c) répétition des phases 3a) et 3b) jusqu'à ce que tous les points soient traités, caractérisé par les phases suivantes du procédé

d) répétition des phases 3a) et 3b) en utilisant le dernier point inclus dans le groupe pour estimer la valeur RK pour la pression de brassard inférieure suivante qui comprend un point et utilisation de la ligne droite de pente prédéterminée m à partir dudit dernier point inclus dans le groupe,

e) répétition des phases 3a), 3b) et 3c) jusqu'à ce que tous les points soient inclus dans un groupe ou un autre, et

4) sélection, comme groupe ne comprenant pratiquement que les points véritables, du groupe qui comprend le plus grand nombre de points.

2. Procédé selon la revendication 1, caractérisé par

5) l'obtention d'une mesure des pressions sanguines systoliques et diastoliques à partir de points adjacents aux extrémités respectives supérieure et inférieure des pressions de brassard pour la phase de sélection 4).

3. Procédé selon la revendication 1 ou 2, caractérisé par

6) l'établissement d'une ligne droite jusqu'à au moins quelques points du groupe sélectionné ne contenant pratiquement que des points véritables de l'opération 4), la pente de ladite ligne droite établissant une mesure de la pente systolique des ondes de pressions sanguines pendant le dégonflement du brassard.

4. Procédé selon l'une des revendications précédentes, caractérisé en ce que la pente prédéterminée m est une pente estimée.

5. Procédé selon la revendication 4, caractérisé en ceci qu'une pente estimée m pratiquement de 1 est employée (la valeur RK étant en unités de ms et la pression de brassard en unités de mmHg).

6. Procédé selon l'une des revendications 1—3 caractérisé en ce que la pente prédéterminée m est établie pendant un cycle d'opérations précédent.

7. Procédé selon l'une des revendications précédentes, caractérisé par la suppression à partir dudit groupe de tous points adjacents à une pression diastolique ayant des valeurs RK supérieures à la plus petite valeur RK à des pressions de brassard moindres que ladite valeur de pression de brassard audit intervalle le plus court.

8. Procédé selon l'une des revendications précédentes, caractérisé par l'utilisation de méthodes des moindres carrés, l'établissement d'une ligne droite jusqu'audit groupe de points et répétition des phases 3(a) à 3(c) utilisant la pente de la ligne droite pour obtenir la valeur estimée $\hat{RK}$.

9. Système pour identifier et enlever les artefacts dans les signaux de bruits de Korotkov comprenant:

1) l'obtention de moyens (36, 48) pour obtenir de façon répétée des mesures de pressions de brassard et de temps d'arrivée de signaux (R) d'ondes R de pointe et de signaux de bruits de Korotkov (K) comprenant des signaux de bruits artefacts (KA) parmi la série de bruits K actifs du dégonflement d'un brassard (30),

2) des détecteurs (52, 66) pour déterminer un tracé de valeurs RK en fonction de points de pressions de brassard dont quelques-uns sont des points véritables et dont d'autres sont des points d'artefacts,

3) un moyen de procéder (44) pour traiter lesdits points afin de les grouper de façon qu'un groupe ne comprenne pratiquement que des points véritables et d'exclure des points d'artefacts comprenant

a) un moyen d'estimation répondant à un point à la pression de brassard la plus élevée pour estimer une valeur $(\hat{RK})$ pour un point véritable à une pression de brassard inférieure suivante qui comprend un point,

b) un moyen de combinaison pour inclure ledit point à la dite pression de brassard inférieure suivant dans un groupe qui comprend ledit point si la différence ($\Delta$RK) entre la valeur RK estimée $(\hat{RK})$ et la valeur RK (RK) à ladite pression de brassard inférieure suivante est au-dessous d'une valeur donnée, autrement excluant ledit point du groupe

c) un moyen de répétition pour répéter les opérations accomplies par des éléments 3a) et 3b) jusqu'à ce qu'il ne reste aucun point non groupé à une pression de brassard inférieure,

d) un autre moyen de répétition pour répéter les opérations accomplies par les éléments 3a), 3b) et 3c) jusqu'à ce que tous les points soient inclus dans un groupe, les points identifiés par l'élément 3d) étant inclus dans un autre groupe de points à chaque répétition des opérations de l'élément 3d) de façon que le moyen d'estimation et/ou le moyen de traitement répondent à une ligne de pente (m) projetée à partir dudit point caractérisé en ce que le moyen de répétition comprend l'utilisation du point ajouté en dernier au groupe pour estimer les valeurs RK $(\hat{RK})$ et que

4) le moyen d'identification identifie le groupe avec le plus grand nombre de points comme un groupe contenant pratiquement tous les points véritables.

10. Système selon la revendication 9, caractérisé en ceci qu'une pente estimée (m) est englobée dans le moyen de procéder (44).

11. Système selon la revendication 9, caractérisé en ceci que la pente (m) comprend une pente de commande obtenue d'un cycle préalable d'opération.

12. Système selon l'une des revendications 9—11 caractérisé en ceci que les moyens d'obtention (36, 48) obtiennent une mesure de pressions sanguines systolique et diastolique de points adjacents aux extrémités supérieure et inférieure respectivement de pressions de brassard du groupe de pratiquement tous les points véritables.

13. Système selon l'une des revendications 9—12, caractérisé en ceci que le moyen d'établissement établit une ligne droite pour au moins quelques-uns des points du groupe de pratiquement tous les points véritables, le pente (m) de ladite ligne droite comprenant une mesure de la pente systolique des ondes de pressions sanguines pendant le dégonflement de brassard.

FIG-1

FIG-2

# FIG-3

# FIG-4

100 START

102 INITIALIZE SYSTEM

A

108 INFLATE CUFF ABOVE SYSTOLIC

110 DEFLATE TO 1st K-SOUND

112 STORE CUFF PRESSURE

114 DETECT & ENTER TIME OF R-PEAK WAVE & ASSOCIATED K-SOUND (& ARTIFACTS)

CALCULATE RK INTERVAL(S) 116

STORE CALCULATED RK INTERVAL(S) 118

DEFLATE CUFF INCREMENTAL AMOUNT 120

122 ABOVE DIASTOLIC PRESSURE? — Yes

No

124 DELETE RK INTERVAL MORE THAN "A" HEART BEATS FROM ADJACENT RK INTERVAL

126 DELETE RK INTERVALS >B or <C

DELETE RK INTERVALS FOLLOWING 1st IF CUFF PRESSURE RISES

128

130 NUMBER OF K-SOUNDS < "D"? — Yes → A

No

132 CONTROL CYCLE? — Yes

No

136 CHAIN POINTS WITH ESTIMATED SLOPE

134 CHAIN POINTS WITH CONTROL SLOPE

138 FIT STRAIGHT LINE TO CHAIN OF MOST POINTS

142 DELETE POINTS TOO FAR FROM LINE

146 DELETE POINTS BELOW INFLECTION POINT NEAR DIASTOLIC PRESSURE

148 DELETE POINTS NEAR DIASTOLIC PRESSURE MORE THAN "E" HEARTBEATS FROM ADJACENT POINT

B

# FIG-5A

3

(B)

FIT STRAIGHT LINE TO REMAINING POINTS — 150

DELETE POINTS TOO FAR FROM LINE — 152

DELETE POINTS BELOW AN INFLECTION POINT NEAR DIASTOLIC PRESSURE — 154

DELETE POINTS NEAR DIASTOLIC PRESSURE WHICH ARE MORE THAN "F" HEART BEATS FROM ADJACENT POINT — 156

FIT STRAIGHT LINE TO REMAINING POINTS — 158

DISPLAY AND/OR RECORD OPPOSITE END POINT SYSTOLIC AND DIASTOLIC PRESSURES — 160

CALCULATE SLOPE OF STRAIGHT LINE AND STORE — 162

TEST OVER? — 164

No → (A)

Yes

END — 166

FIG-5B

# FIG-6

FROM STEP 132

170 — ANY UNGROUPED POINTS? —NO→ GO TO STEP 138

YES

172 — STORE UNGROUPED POINT AT HIGHEST CUFF PRESSURE WITH GROUP "X"

174 — ANY UNGROUPED POINTS? —NO→ GO TO STEP 138

YES

176 — FROM LAST-ENTERED POINT, ESTIMATE RK INTERVAL FOR NEXT LOWER CUFF PRESSURE WHICH INCLUDES AN UNGROUPED POINT

178 — COMPUTE $\Delta RK = \widehat{RK} - RK$

180 — $\Delta RK > G$? —YES

NO

182 — STORE RK INTERVAL POINT FROM STEP 178 WITH GROUP "X"

186 — INCREMENT "X" ←NO— MORE UNGROUPED POINTS AT LOWER CUFF PRESSURE?

184

YES

## FIG-7

$(\widehat{RK}_{n+1}, C_{n+1})$

$(RK_{n+2}, C_{n+2})$

$(\widehat{RK}_n, C_n)$

C1

$(RK_{n-1}, C_{n-1})$

$(RK_{n+3}, C_{n+3})$

$\Delta RK_n = \widehat{RK}_n - RK_n$

C2

$(RK_n, C_n)$

$(RK_{n+2}, C_{n+2})$

$(RK_{n+1}, C_{n+1})$

RK INTERVAL →

Estimated RK Interval

×Chain 1
+Chain 2

• RK Interval vs cuff pressure points

CUFF PRESSURE →

## FIG-8